Europäisches Patentamt
European Patent Office
Office européen des brevets

(19)

(11) Publication number: 0 273 727
A2

# (12) EUROPEAN PATENT APPLICATION

(21) Application number: 87311421.9

(22) Date of filing: 23.12.87

(51) Int. Cl.[4]: C 07 K 9/00
A 61 K 37/02, A 23 K 1/00

(30) Priority: 30.12.86 US 948175

(43) Date of publication of application:
06.07.88 Bulletin 88/27

(84) Designated Contracting States:
AT BE CH DE ES FR GB GR IT LI LU NL SE

(71) Applicant: SMITHKLINE BECKMAN CORPORATION
One Franklin Plaza P O Box 7929
Philadelphia Pennsylvania 19103 (US)

(72) Inventor: Christensen IV, Siegfried Benjamin
2305 Naudain Street
Philadelphia PA 19146 (US)

Chung, Sung Kee
Postech Fac. Apt. 2-503 Pohang Inst. of Science & Technology Pohang 680 (KR)

Jeffs, Peter Walter
1263 Club House Road
Gladwyne Pennsylvania 19035 (US)

(74) Representative: Waters, David Martin, Dr.
Smith Kline & French Laboratories Ltd. Patent Department Mundells
Welwyn Garden City Hertfordshire AL7 1EY (GB)

Claims for the following Contracting States: ES + GR.

## (54) Glycosylated glycopeptides.

(57) Gylcosylated glycopeptide antibiotics having the formula I

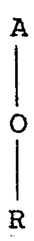

(I)

in which A is the remaining portion of a glycopeptide antibiotic of the vancomycin/ristocetin class or a chemical derivative thereof, and O is the phenolic oxygen of the central (B) ring of the triaryl ether region of the glycopeptide antibiotic; and R is a monosaccharide or disaccharide or a deoxy or amino variant thereof; provided that R is not the natural sugar species attached at this site on the glycopeptide core, and R is not glucosyl when A is the remaining portion of vancomycin, A51568A, M43D, or A51568B and their salts are useful new antibiotics. A process for producing compounds of formula (I) or a chemical derivative thereof, or a pharmaceutically acceptable salt thereof. These derivatives are useful for treating or preventing infection in an animal by gram-positive bacteria and also increase feed-utilization efficiency, promote growth in domestic animals and increase propionate production in lactating ruminants.

EP 0 273 727 A2

**Description**

# GLYCOSYLATED GLYCOPEPTIDES

## Field of the Invention

This invention relates to selectively glycosylated glycopeptide antibiotics and the process for preparing them.

## Background of the Invention

The vancomycin/ristocetin, class of glycopeptide antibiotics are amorphous, amphoteric, strongly laevo-rotatory compounds of relatively high molecular weight. Structurally, they comprise a heptapeptide aglycone core having phenolic amino acids and, usually, one or more peripheral carbohydrate moieties. See, Williams et al., Topics in Antibiotic Chemistry, Volume 5, pages 119-158. Known members of this class include vancomycin (McCormick et al., U.S. Patent 3,067,099), ristocetin (Philip et al., U.S. Patent 2,990,329), A35512 (Michel et al., U.S. Patent 4,083,964), avoparcin (Kunstmann et al., U.S. Patent 3,338,786 and Debono, U.S. Patent 4,322,343), teicoplanin (Bardone et al., J. Antibiot., Volume 31, page 170, 1978), actaplanin (Raun, U.S. Patent 3,816,618, Boech et al., U.S. Patent No. 4,537,715), AAD-216 ("ardacin") (Bowie et al., U.S. Patent No. 4,548,974), A477 (Raun et al., U.S. Patent 3,928,571), OA7653 (Nishida et al., U.S. Patent 4,378,348), AM 374 (Kunstmann et al., U.S. Patent 3,803,306), K288 (J. Antibiotics, Series A, Volume 14, page 141 (1961), also known as actinoidin), teichomycin (Borghi et al., U.S. Patent 4,542,018, Malabarba et al., The journal of Antibiotics, Vol. XXXVII, No. 9, p 988-999, Barna et al., The Journal of Antibiotics, Vol. XXX011, No. 9, p 1204-1208), desvancosaminyl and des (vancosaminyl-O-glucosyl) glycopeptides (Nagarajan, U.S. Patent 4,552,701), AAJ-271, (Carr et al. copending application EPA No. 87306193.1 incorporated herein by reference), A 33512B (U.S. Patent No. 4,029,769), A 41030 factors a-g (U.S. Patent No. 4,537,770) and CWI-785 (copending EPA No. 87306558.5 incorporated by reference herein), A51568 factors A and B (Hoehn et al., U.S. Patent No. 4,495,179), and M43D (Merkel, U.S. Patent No. 4,547,488).

The glycopeptide antibiotics exhibit antibacterial activity, some having therepeutic uses against gram-positive organisms including methicillin-resistant strains. These strains currently cannot be treated with β-lactam antibiotics, including the newer β-lactamase-resistant cephalosporins. Infections by these pathogens is a serious problem. For example the compounds of this invention may be used to treat staphylococcal endocarditis, osteomyelitis, pneumonia, septicemia, soft tissue infection, staphylococcal enterocolitis and antibiotic-associated pseudomembranous colitis produced by C. difficile. They may also be used for prophylaxis for hip and heart surgery, prophylaxis against bacterial endocarditis and S. aureus infections in hemodialysis patients.

Many glycopeptides have also been demonstrated to increase animal feed utilization efficiency and, therefore, to be useful to promote animal growth, to improve milk production in ruminants and to treat and to prevent ketosis in ruminants. For example, Reynolds et al., GB2137087A, disclose the use of avoparcin to improve milk production; Raun et al., U.S. Patent 3,928,571 disclose the use of actaplanin, avoparcin (A471), vancomycin and ristocetin to promote growth and to prevent and to treat ketosis; Hamill et al., U.S. Patent 3,952,095, disclose the use of actaplanin to promote growth; and Ingle et al., U.S. Patent 4,206,203 disclose use of avoparcin to prevent and to treat ketosis.

New improved antibiotics are continually in demand, particularly for the treatment of human diseases. Increased potency, expanded spectrum of bacterial inhibition, increased in vivo efficacy, and improved pharmaceutical properties, such as greater oral absorption, higher blood or tissue concentration, longer in vivo half life, and more advantageous rate of route of excretion and rate or pattern of metabolism are some of the goals for improved antibiotics.

In addition to searching for such new compounds in nature, chemical derivatives of existing compounds are being made. An early approach was hydrolysis to remove one or more carbohydrate moieties, (e.g. Chan et al., U.S. Patent 4,521,335 and Nagarajan et al., U.S. Patent No. 4,552,701). Another approach described in Debono, U.S. Patent 4,497,802 is to acylate the amine terminus of the glycopeptide nucleus.

## Summary of the Invention

In one aspect, the invention comprises new selectively glycosylated glycopeptide antibiotics. Representatives of these compounds are O-(β-D-glucopyranosyl)-HPB-4′, O-(2-acetamido-2-deoxy-β-D-glucopyranosyl)-HPB-4′, O-[2-(methoxycarbonyl)amino-2-deoxy-β-D-glucopyranosyl]-HPB-4′, O[2-(1-methylethoxycarbonyl)-amino-2-deoxy-β-D-glucopyranosyl]-HPB-4′, and O-[2-(ethoxycarbonyl)amino-2-deoxy-β-D-glucopyranosyl]-HPB-4′. HPB-4′ is a pseudoaglycone of a CWI-785 antibiotic of the R configuration at the α-carbon (6-1′) of the terminal N-methyl-p-hydroxyglycine residue.

In yet other aspects, the invention is a process for preparing the antibiotics of the invention, an antibacterial composition comprising such antibiotics, compounds for use as medicaments, inparticular for use in treating or preventing gram-positive bacterial infections in an animal (including man), an animal feed composition comprising such antibiotics to increase propionate production in the rumen or ceum of a meat or milk producing animal, an animal feed premix containing such antibiotics, a method of improving the growth rate of a meat producing animal by administration of such antibiotics, a method of improving the efficiency of feed

utilization in a meat or milk producing animal by administration of such antibiotics and a method for improving milk production in a lactating ruminant by administration of such antibiotics.

These and other aspects described herein below are considered embodiments of the same invention and are fully disclosed herein.

Detailed Description

The antibiotics of this invention are chemically prepared glycosylated derivatives of other glycopeptide antibiotics of the vancomycin/ristocetin class. They are represented by formula I:

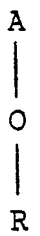

(I)

wherein A is the remaining portion of a gylcopeptide antibiotic of the vancomycin/ristocetin class or a chemical derivative thereof, O is the central phenolic oxygen of the central (B) ring of the triaryl ether region of the glycopeptide antibiotic; and R is a monosaccharide, or disaccharide or a deoxy or amino variant thereof; provided that R is not the natural sugar species attached at this site on the glycopeptide core, and R is not glucosyl when A is the remaining portion of vancomycin, A 51568A, M 34D, or A 51568B. The salts of the compounds of formula I are also part of this invention.

A can be the remaining portion of any glycopeptide antibiotic of the vancomycin/ristocetin class or chemical derivative thereof which has substantially the core structure found in formula II, provided that if a carbohydrate moiety (Sug) is present on the phenolic oxygen of the central (B) ring it is first hydrolyzed off so that the B-4 phenol is free before glycosylation is performed.

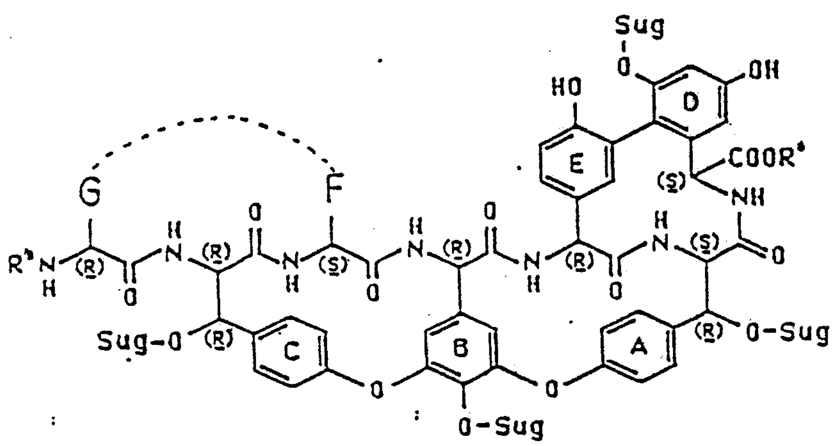

(II)

Sug is a Carbohydrate or H

$R^1$ is H or Me

F is the side chain of asparagine, when G is the side chain of N-methylleucine (i.e., vancomycin); or

F and G together constitute a diphenylether fragment (i.e., ristocetin A, actaplanin A 35512B and teicoplanin); or

F and G are each an aromatic residue (i.e., actinioden and avoparcin); or

G is an oxygenated aromatic residue such as phenol, when F is the side chain of methionine (i.e., CWI-785 glycopeptides).

By the remaining portion of a glycopeptide antibiotic of the vancomycin/ristocetin class or a chemical derivative thereof we mean the complete structure of the antibiotic or a chemical derivative thereof but excluding the central phenolic oxygen of the central (B) ring of the triaryl ether region thereof and excluding the phenolic oxygen substituent, i.e. hydrogen or a carbohydrate. For example the remaining portion of a glycopeptide antibiotic of the formula (II) is :

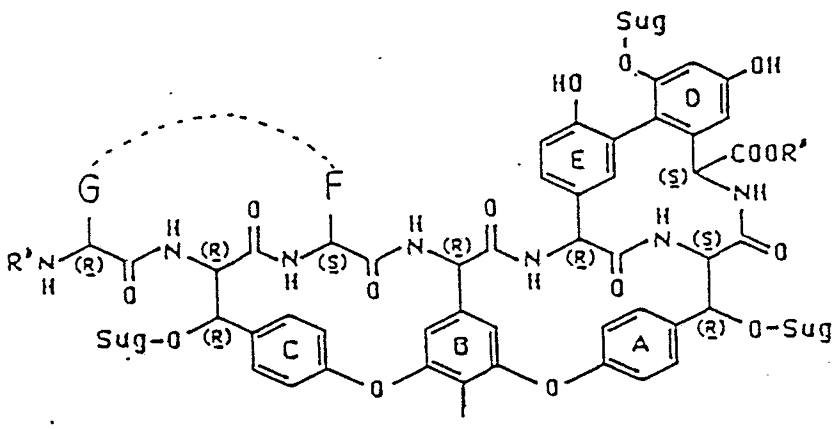

(II)

wherein $R^1$, Sug, F and G are as hereinbefore defined.

Exemplary of glycopeptide antibiotics of the vancomycin/ristocetin class which are included in formula II are vancomycin, ristocetin, actaplanin, A35512B, teicoplanin, AAJ-271 glycopeptides, A41030 factors a, b, c, d, e, f, g, CWI-785 glycopeptides, actinodin, AAD-216 glycopeptides, avoparcin M43A, B, C, D, A51568A and B, AM374, A477, OA7653, their aglycones, pseudoaglycones, N-acyl, and ester derivatives provided that any carbohydrate on the phenolic oxygen of the central B ring is first hydrolyzed off before glycosylation is performed.

Chemical derivatives include, e.g., hydrolysis products such as aglycones and pseudoaglycones, and synthetic derivatives such as those disclosed in U.S. Patent 4,497,802 (N-acylglycopeptides), U.S. Patent 4,552,701 (desvancosaminyl and des(vancosaminyl-O-glucosyl)-glycopeptides) or European Patent 182-157A (ester derivatives of teicoplanin pseudoaglycone).

A preferred subgroup of formula I compounds are those wherein A id the remaining portion of AAD-216 aglycone, AAD-216 mannosylaglycone, HPB-4′, CWI-785B′ aglycone, HPB-3′, AAJ-271 mannosylaglycone, and vancomycin aglycone.

Especially preferred are the compounds in which A is the remaining portion of HPB-4′.

R is any sugar, i.e., a monosaccharide, or disaccharide or their deoxy or amino variants.

Exemplary of R are pyranosyl or furanosyl monosaccharides including glucosyl; amino-deoxy-glucosyl; diamino-dideoxy-glucosyl, N-acylamino-deoxy-glucosyl, or compounds of formula III:

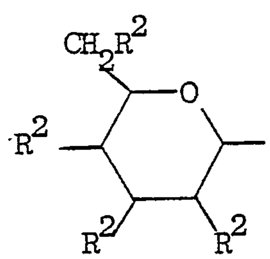

(III) .

wherein

$R^2$ is OH, $NR^3R^4$, or $NR^3$-$(CH_2)_s$-Wp-$(CH_2)_qR^5$;

$R^3$ is H or $C_1$-$C_4$ alkyl;

$R^4$ is H,

$$-\overset{O}{\overset{\|}{C}}-(CH_2)_mY_p-(CH_2)_n-R^5; \text{ or}$$

$$-\overset{O}{\overset{\|}{\underset{O}{\underset{\|}{S}}}}-R^5;$$

p is 0 or 1;
m and n are integers from 0 to 4;
s is an integer from 2 to 4;
q is an integer from 1 to 4;
$R^5$ is H, $C_1$-$C_4$-alkyl, $C_3$-$C_8$-cycloalkyl, $C_4$-$C_8$-cycloalkenyl, or phenyl;
W is $CH_2$, -C≡C, -CH=CH-, -C($CH_3$)=CH-, -CH=C($CH_3$)-or -C($CH_3$)=C($CH_3$);
Y is $CH_2$, O, -N($C_1$-$C_4$ alkyl)-, -NH-, -C≡C-, -CH=CH-, -C($CH_3$)=CH-, -CH=C($CH_3$)- or -C($CH_3$)=C($CH_3$)-; and wherein $R^5$ and the connecting alkyl groups $-(CH_2)_m$, $-(CH_2)_n$-, $(CH_2)_s$- and $-(CH_2)_q$- are optionally substituted by one or two halo, methyl, ethyl, methoxy, amino, N-protected-amino, methylamino, dimethylamino, acetamido, or hydroxyl groups, provided that $R^5$ is not H when Y is O, p is 1, m is O, and n is O; and provided that at least two $R^2$ groups are OH; or

R is mannosyl, O-2-α-L- (ristosaminyl)-O-β-D-glucose, O-2-α-L- (acosaminyl)-O-β-D-glucose or O-2-α-L-(vancosaminyl)-O-β-D-glucose so long as the natural sugar species is not reacted with the parent glycopeptide core and R is not glucosyl when A is the remaining portion of vancomycin, A51568A, M43D, or A50568B.

Especially preferred are the compounds in which R is glucosyl, 2-acetamido-2-deoxy-D-glucosyl, 2-(methoxycarbonyl)amino-2-deoxy-D-glucosyl, 2-(1-methylethoxycarbonyl)amino-2-deoxy-D-glucosyl, and 2-(ethoxycarbonyl)-amino-2-deoxy-D-glucosyl.

This invention also provides for a process for preparing a compound of the formula A-O-R or a chemical derivative thereof, or a pharmaceutically acceptable salt thereof, wherein A is the remaining portion of a glycopeptide antibiotic of the vancomycin/ristocetin class or a chemical derivative thereof, and O is the phenolic oxygen of the central (B) ring of the triaryl ether region of the gylcopeptide antibiotic and R is a saccharide selected from: monosaccharides, disaccharides, and their deoxy and amino variants; which process comprises reacting a protected α-glycosyl halide with an alkaline solution of a glycopeptide antibiotic of the formula A-O-H or a chemical derivative thereof, wherein A and O are as hereinbefore defined, for a time sufficient to produce the protected glycosylated glycopeptide antibiotic as an intermediate, and thereafter;

i) deprotecting the functionalities of the added glycosy group by methods known to those skilled in the art,
ii) optionally forming a chemical derivative thereof,
iii) optionally forming a pharmaceutically acceptable salt thereof,

The reaction is suitable only for the production of aryl glycosides, and proceeds with inversion of configuration at the anomeric carbon of the α-glucosyl halide.

An unexpected and surprisingly unique feature of the present process is that the aglycones or pseudoaglycones of glycopeptides which posses four to six potential phenolic glycosylation sites react with excess reagents to provide as the major product a β-glycoside with B4-phenol attachment.

A variety of bases may be used in the reaction. Examples include lithium hydroxide, sodium hydroxide, potassium hydroxide, potassium carbonate, ammonium hydroxide, triethylamine and barium hydroxide. Potassium hydroxide is preferred.

The organic cosolvent may be any solvent in which the reactants are soluble. Example are: aceton, DMF, DMSO, isopropanol, acetonitrile, methanol, tetrahydrofuran or dioxane. Acetone is the preferred solvent.

The protecting group for the oxygens of the glycosyl halide may be oxygen esters or carbonate protecting groups which are stable enough in the basic solution to permit the initial glycosylation and subsequent ease of removal. Examples are O-acetyl and O-benzoyl. The preferred protecting group is O-acetyl.

Examples of suitable nitrogen protecting groups are trifluoroacetyl and trichloroacetyl. Trifluoroacetyl is preferred.

Examples of basic depotection conditions which may be used and are standard in the art include (a) a 25-50% solution of ammonium hydroxide in methanol, (b) concentrated ammonium hydroxide, (c) ammonia (anhydrous) saturated methanol, (d) methanolic sodium methoxide, (e) aqueous piperidine and (f) aqueous potassium carbonate. The use of 25-50% ammonium hydroxide in methanol is preferred as it gives the fastest

and cleanest deprotec tion of both O-acetyl and N-trifluoroacetyl protecting groups.

The preferred general process for preparing the compounds of formula I is to add an acetone solution of an acetylated glycosyl halide (about 2 to about 10 equivalents, with 2-5 equivalents preferred) rapidly to a solution of the glycopeptide in aqueous acetone containing about 2 to about 10 equivalents (3-6 equivalents preferred) of potassium hydroxide. Superior results are obtained when the final glycopeptide concentration is about 0.02-0.05 M, however, this is not critical. The solution is stirred for one to two hours at room temperature. After the solvents are removed in vacuo, the residue is treated with concentrated ammonium hydroxide in methanol (1:1) for about 1 to about 30 hrs at room temperature to cleave the protecting groups.

Chromatographic purification is done using a D-Ala-D-Ala affinity gel column (as outlined in U.S. Patent No. 4,667,024 and incorporated by reference hererein) and reversed phase HPLC.

The isolated major product of each reaction was characterized initially on the basis of its reversed phase HPLC profile. Mass spectral (MS) data were obtained using a mass spectrometer equipped with a standard FAB source in a matrix of monothioglycerol containing oxalic acid. Accurate mass measurements were obtained by peak matching of the protonated molecular ion at a resolution of 10,000 against a con-introduced oligopeptide of known structure and molecular weight (1151.538). Nominal mass measurements for the low-resolution FAB MS of each major product revealed the presence of only one additional sugar relative to the starting compound. Accurate mass measurements from high resolution FAB MS provided elemental composition consistent with expectation for the proposed structure. Experimentally determined isoelectric points (pI) were also consistent with expection. Mass spec, pI and E1% data are given in Example 2 and 5-25.

The site and stereochemistry of the added sugar was determined by analysis of 500 mHz $^1$H NMR spectral data of representative products. Chemical shift assignments for individual resonances were made on the basis of two-dimensional correlation spectroscopy (COSY) by analogy to the parent glycopeptide and/or its aglycone. This was followed by two dimensional nuclear Overhauser spectroscopy (NOSEY) as described by Jeffs et al., J.A.C.S. 1986, 3063-3075. The spectral patterns established a B-4, β-linked sugar.

The preferred parent antibiotics used as starting materials in the process of this invention are all members of the vancomycin/ristocetin gylcopeptide antibiotic group. The AAD-216 antibiotics are described in U.S. Patent No. 4,548,974. The AAJ-271 aglycones are described in copending EPA No. 87306193.1 incorporated by reference herein. HPB-4′, HPB-4″, HPB-3′, HPB-3″, HPB-2M and CWI-785B-aglycone are described in copending EPA No. 87306558.5 incorporated by reference herein. Vancomycin aglycone is described by F. J. Marshall in J. Med. Chem., 1965, Vol. 8, p. 18-22.

The glycosylated glycopeptide antibiotics may serve as starting materials for further chemical modification. For example, the glycosylated glycopeptides in which an amino-deoxy sugar has been added may be further modified by acylation or reductive amination of the amino group of the introduced sugar. These procedures are described in U.S. Patent Nos. 4,497,802 and 4,482,481. Introduction of the acyl or alkyl group on the free amine of the added sugar, rather than direct glycosylation of the nucleus using a protected halo sugar reagent containing the N-acyl or N-alkyl chain, would allow production of those compounds difficult to prepare by direct glycosyl ation. Since any amine sites originally present in the nucleus of the glycopeptide would also be acylated or alkylated, these sites must be protected prior to glycosylation, preferably with a t-butyloxycarbonyl protecting group. After glycosylation of the N-protected glycopeptide and subsequent deprotection of the oxygen and amine group(s) of the added sugar, acylation or reductive amination of the free amino group of the added sugar may be done. Finally, any N-protecting group on the nuclear amine may be removed.

The structures of HPB-4′, HPB-3′ and CWI-785B′ aglycone and their glycosylated derivatives are shown in formulas 2a to 2q.

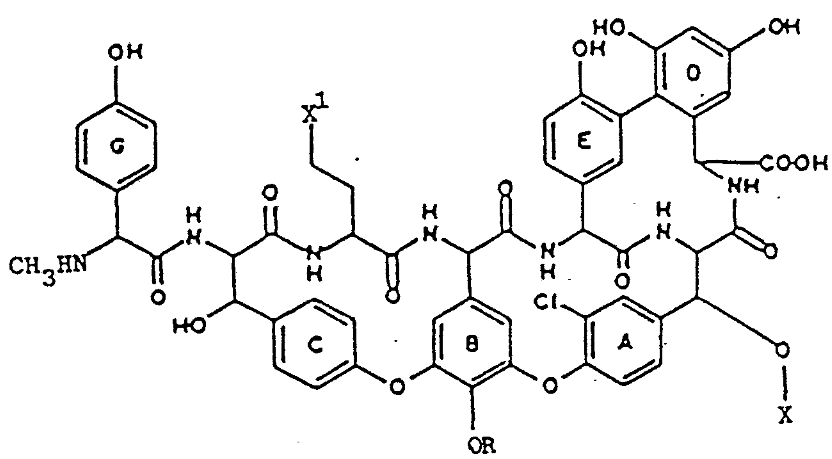

| | COMPOUND | X | $X^1$ | R |
|---|---|---|---|---|
| 2a | HPB-4 | ristosamine | $-SCH_3$ | H |
| 2b | HPB-3' | ristosamine | $-\overset{O}{\overset{\|}{S}}CH_3$ | H |
| 2c | CWI-785B' aglycone | H | $-SCH_3$ | H |
| 2d | O-(ß-D-glucopyranosyl)-HPB-4' | ristosamine | $-SCH_3$ | D-glucose |
| 2e | O-(ß-D-glucopyranosyl)-HPB-3 | ristosamine | $-\overset{O}{\overset{\|}{S}}CH_3$ | D-glucose |
| 2f | O-(2-amino-2-deoxy-ß-D-glucopyranosyl)-CWI-785B' aglycone | H | $-SCH_3$ | 2-amino-2-deoxy-D-glucose |
| 2g | O-(2-amino-2-deoxy-ß-D-glucopyranosyl)-HPB-4' | ristosamine | $-SCH_3$ | 2-amino-2-deoxy-D-glucose |
| 2h | O-[2-(benzyloxycarbonyl)amino-2-deoxy-ß-D-glucopyranosyl]-HPB-4' | ristosamine | $-SCH_3$ | 2-(benzyloxycarbonyl)amino-2-deoxy-D-glucose |
| 2i | O-(2-butanamido-2-deoxy-ß-D-glucopyranosyl)-HPB-4' | ristosamine | $-SCH_3$ | 2-butanamido-2-deoxy-D-glucose |
| 2j | O-[2-(3-methylbutanamido)-2-deoxy-ß-D-glucopyranosyl]-HPB-4' | ristosamine | $-SCH_3$ | 2-(3-methylbutanamido)-2-deoxy-D-glucose |
| 2k | O-[2-(2-methylpropoxycarbonyl)-amino-2-deoxy-ß-D-glucopyranosyl]-HPB-4' | ristosamine | $-SCH_3$ | 2-(2-methylpropoxycarbonyl)amino-2-deoxy-D-glucose |
| 2l | O-[2-(1-methylethoxycarbonyl)-amino-2-deoxy-ß-D-glucopyranosyl)-HPB-4' | ristosamine | $-SCH_3$ | 2-(1-methylethoxycarbonyl)amino-2-deoxy-D-glucose |
| 2m | O-(2-acetamido-2-deoxy-ß-D-glucopyranosyl)-HPB-4' | ristosamine | $-SCH_3$ | 2-acetamido-2-deoxy-D-glucose |
| 2n | O-[2-(propoxycarbonyl)amino-2-deoxy-ß-D-glucopyranosyl]-HPB-4' | ristosamine | $-SCH_3$ | 2-(propoxycarbonyl)amino-2-deoxy-D-glucose |
| 2o | O-[2-(methoxycarbonyl)amino-2-deoxy-ß-D-glucopyranosyl]-HPB-4' | ristosamine | $-SCH_3$ | 2-(methoxycarbonyl)amino-2-deoxy-D-glucose |
| 2p | O-(2-propanamido-2-deoxy-ß-D-glucopyranosyl)-HPB-4' | ristosamine | $-SCH_3$ | 2-propanamido-2-deoxy-D-glucose |
| 2q | O-[2-(ethoxycarbonyl)amino-2-deoxy-ß-D-glucopyranosyl]-HPB-4' | ristosamine | $-SCH_3$ | 2-(ethoxycarbonyl)amino-2-deoxy-D-glucose |

The structures of AAD-216 aglycone (HP-3), AAD-216-mannosylaglycone (HP-4) AAJ-271 mannosylaglycone and their glycosylated derivatives are shown in formula 3a - 3k.

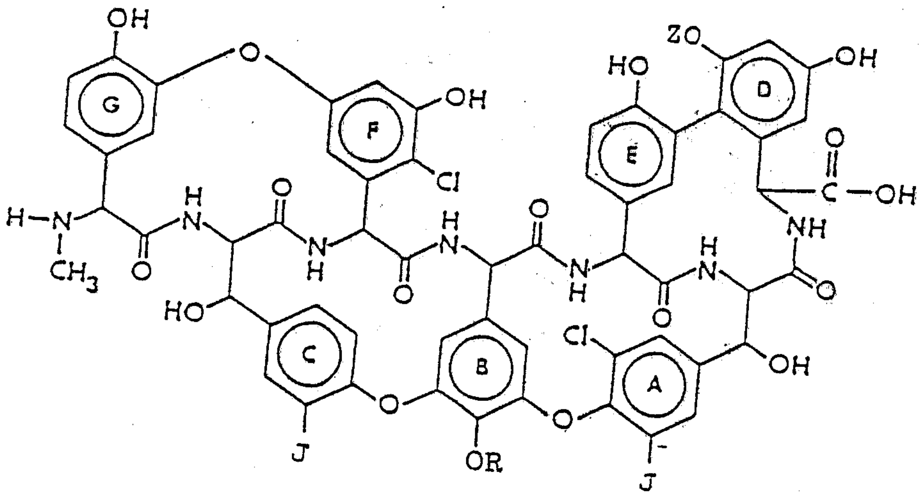

| | Compound | Z | R | J |
|---|---|---|---|---|
| 3a | HP-3 | H | H | Cl |
| 3b | HP-4 | D-mannose | H | Cl |
| 3c | O-(ß-D-glucopyranosyl)-HP-3 | H | D-glucose | Cl |
| 3d | O-(ß-D-mannopyranosyl)-HP-3 | H | D-mannose | Cl |
| 3e | O-(2-acetamido-2-deoxy-ß-D-glucopyranosyl)-HP-3 | H | 2-acetamido-2-deoxy-D-glucose | Cl |
| 3f | O-(2-amino-2-deoxy-ß-D-glucopyranosyl)-HP-3 | H | 2-amino-2-deoxy-D-glucose | Cl |
| 3g | O-(6-amino-6-deoxy-ß-D-gluco-pyranosyl-HP-3 | H | 6-amino-6-deoxy-D-glucose | Cl |
| 3h | O-(6-acetamido-6-ueoxy-ß-D-gluco-pyranosyl-HPB-3' | H | 6-acetamido-6-deoxy-D-glucose | Cl |
| 3i | O-(ß-D-maltosyl)-HP-3 | H | D-maltose | Cl |
| 3j | O-(2-acetamido-2-deoxy-ß-D-glucopyranosyl)-HP-4 | D-mannose | 2-acetamido-2-deoxy-D-glucose | Cl |
| 3k | O-(6-amino-6-deoxy-ß-D-glucopyranosyl)-AAJ-271 mannosyl aglycone | D-mannose | 6-amino-6-deoxy-D-glucose | H |

The structures of vancomycin aglycone and its glycosylated derivatives are shown in formula 4a-4c.

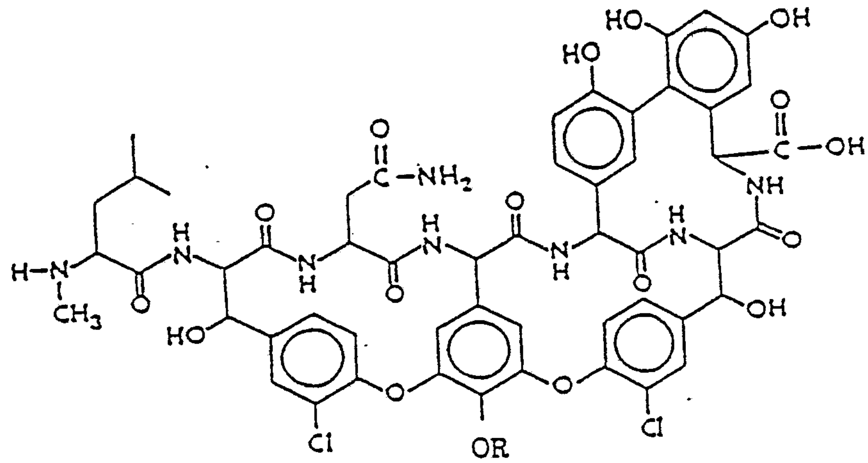

| | Compound | R |
|---|---|---|
| 4a | vancomycin aglycone | H |
| 4b | O-(2-amino-2-deoxy-ß-D-glucopyranosyl)-vancomycin aglycone | 2-amino-2-deoxy-D-glucose |
| 4c | O-(6-amino-6-deoxy-ß-D-glucopyranosyl)-vancomycin aglycone | 6-amino-6-deoxy-D-glucose |

The antibiotics of this invention can be converted to physiologically acceptable salts by techniques well-known in the art. Such salts are formed with strong or moderately strong organic or inorganic acids. For example, the antibiotic is reacted with such acid in an aqueous miscible solvent such as ethanol with isolation of the salt by precipitation such as with excess ethyl ether or chloroform with the described salt separating directly or by removing the solvent. Exemplary of salts included in this invention are acetate, oxalate, methan, sulfonate ethane sulfonate, benzene sulfonate, tartrate, citrate, salicylate, acetate, propionate, hydrochloride, hydrobromide, sulfate, toluene-sulfonic, phosphate and nitrate salts. The phosphate, methane sulfonate, and acetate salts are preferred.

The antibiotics of this invention, and the salts thereof, all exhibit antibacterial activity in in vitro and in vivo activity assays against gram-positive organisms and can be used, therefore, to prevent or to treat infection in a human or animal by, for example, Staphylococcus (including beta-lactam resistant strains), Streptococcus and Clostridium species.

Representative results of standard microtiter assays are reported in Table 1 which follows, as the minimum inhibitory concentration of antibiotic ( g/ml).

In Table 1, test organisms 1-5 were different strains of Staphylococcus aureus; 6, 8, 11, 13 and 14 were different strains of Staphylococcus epidermidis; 7 was a Staphylococcus haemolyticus; 9 and 10 were different strain of Streptococcus faecalis; and 12 was a Staphylococcus saprophyticus.

Table 1

| Antibiotic | Test Organism 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| O-(β-D-glucopyranosyl)-AAD-216 aglycone | 1.6 | - | 1.6 | - | 3.1 | 6.3 | 12.5 | - | 1.6 | 1.6 | - | - | - | - |
| O-(β-D-mannopyranosyl)-AAD-216 aglycone | 1.6 | - | 0.8 | - | 1.6 | 6.3 | 25 | - | 1.6 | 1.6 | - | - | - | - |
| O-(2-acetamido-2-deoxy-β-D-glucopyranosyl)-AAD-216 aglycone | 3.1 | 3.1 | 1.6 | 1.6 | 1.6 | 6.3 | 12.5 | 6.3 | 3.1 | 3.1 | 12.5 | 3.1 | 6.3 | 3.1 |
| O-(2-amino-2-deoxy-β-D-glucopyranosyl)-AAD-216 aglycone | 3.1 | 1.6 | 1.6 | 1.6 | 1.6 | 6.3 | 6.3 | 6.3 | 1.6 | 1.6 | 6.3 | 6.3 | 6.3 | 6.3 |
| O-(6-amino-6-deoxy-β-D-glucopyranosyl)-AAD-216 aglycone | 0.8 | 0.8 | 0.8 | 1.6 | 0.8 | 3.1 | 3.1 | 3.1 | 0.8 | 0.8 | 3.1 | 1.6 | 1.6 | 3.1 |
| O-(6-acetamido-6-deoxy-β-D-glucopyranosyl)-AAD-216 aglycone | 3.1 | 3.1 | 1.6 | 3.1 | 3.1 | 12.5 | 12.5 | 12.5 | 3.1 | 3.1 | 25 | 6.3 | 12.5 | 3.1 |
| O-(β-D-maltosyl)-AAD-216 aglycone | 6.3 | - | 1.6 | - | 6.3 | 12.5 | 25 | - | 1.6 | 1.6 | - | - | - | - |
| O-(2-acetamido-2-deoxy-β-D-glucopyranosyl)-AAD-216 mannosylaglycone | 12.5 | 12.5 | 6.3 | 12.5 | 12.5 | 50 | 100 | 100 | 6.3 | 6.3 | 100 | 25 | 50 | 50 |
| O-(2-amino-2-deoxy-β-D-glucopyranosyl)-HPB-4' | 1.6 | - | 0.8 | - | 1.6 | 3.1 | 12.5 | - | 0.8 | 0.8 | - | - | - | - |
| O-(2-amino-2-deoxy-β-D-glucopyranosyl) CWI-785B' aglycone | 1.6 | - | 1.6 | - | 3.1 | 3.1 | 6.3 | - | 3.1 | 3.1 | - | - | - | - |
| O-(β-D-glucopyranosyl)-HPB-3' | 6.3 | - | 3.1 | - | 3.1 | 6.3 | 6.3 | 6.3 | 1.6 | 1.6 | - | - | - | - |
| AAD-216 aglycone | 0.8 | 0.4 | 0.4 | 0.4 | 0.8 | 0.4 | 3.1 | 3.1 | 1.6 | 1.6 | 1.6 | 1.6 | 0.8 | 0.8 |

| Antibiotic | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| AAD-216 mannosyl-aglycone | 1.6 | 1.6 | 0.8 | 1.6 | 1.6 | 12.5 | 25 | 6.3 | 0.8 | 0.8 | 25 | 3.1 | 6.3 | - |
| O-(6-amino-6-deoxy-β-D-glucopyranosyl)-AAJ-271 mannosyl aglycone | 12.5 | - | - | 12.5 | 12.5 | 12.5 | 25 | 50 | 6.3 | 6.3 | 50 | 12.5 | - | - |
| HPB-4' | 0.8 | 0.8 | 0.4 | 0.8 | 0.8 | 1.6 | 3.1 | 1.6 | 0.4 | 0.4 | 3.1 | 1.6 | 1.6 | - |
| CWI-785B' aglycone | 3.1 | - | 3.1 | - | 3.1 | 3.1 | 6.3 | 6.3 | 0.8 | 0.8 | - | - | - | - |
| HPB-3' | 0.8 | 1.6 | 0.8 | 1.6 | 0.8 | 1.6 | 3.1 | 1.6 | 1.6 | 0.8 | 3.1 | 1.6 | 0.8 | 1.6 |
| Vancomycin | 3.1 | 1.6 | 1.6 | 3.1 | 1.6 | 3.1 | 3.1 | 3.1 | 3.1 | 3.1 | 3.1 | 3.1 | 3.1 | 3.1 |

Table 2, which follows, shows MIC data against an expanded panel. The number of strains tested is shown in parentheses below each organism. The $MIC_{50}$ is reported in µg/ml.

Table 2

Test Organisms

| Antibiotic | S. Aureus (10) | S. Epidermidis (20) | St. faecalis (10) |
|---|---|---|---|
| 2d | | | |
| $MIC_{50}$ | 1.6 | 6.3 | 1.6 |
| RANGE | (1.6-6.3) | (3.1-12.5) | (0.8-1.6) |
| 2m | | | |
| $MIC_{50}$ | 1.6 | 6.3 | 0.8 |
| RANGE | (1.6-3.1) | (1.6-12.5) | (0.8-1.6) |
| 2n | | | |
| $MIC_{50}$ | 3.1 | 6.3 | 1.6 |
| RANGE | (3.1-6.3) | (3.1-25) | (0.8-3.1) |
| 4b | | | |
| $MIC_{50}$ | 3.1 | 6.3 | 1.6 |
| RANGE | (3.1-6.3) | (6.3-12.5) | (1.6-3.1) |
| 2j | | | |
| $MIC_{50}$ | 3.1 | 6.3 | 1.6 |
| RANGE | (3.1-6.3) | (3.1-12.5) | (0.8-1.6) |
| 2l | | | |
| $MIC_{50}$ | 3.1 | 3.1 | 1.6 |
| RANGE | (3.1-6.3) | (1.6-12.5) | (0.8-1.6) |
| 2k | | | |
| $MIC_{50}$ | 3.1 | 6.3 | 0.8 |
| RANGE | (1.6-6.3) | (3.1-25) | (0.8-1.6) |
| 2h | | | |
| $MIC_{50}$ | 3.1 | 6.3 | 0.8 |
| RANGE | (1.6-3.1) | (3.1-25) | (0.8-1.6) |
| 2i | | | |
| $MIC_{50}$ | 3.1 | 6.3 | 0.8 |
| RANGE | (1.6-6.3) | (3.1-12.5) | (0.8-1.6) |
| 2o | | | |
| $MIC_{50}$ | 3.1 | 6.3 | 0.8 |
| RANGE | (1.6-6.3) | (3.1-12.5) | (0.8-1.6) |

Table 2 - Cont'd

Test Organisms

| Antibiotic | S. Aureus (10) | S. Epidermidis (20) | St. faecalis (10) |
|---|---|---|---|
| 2p | | | |
| $MIC_{50}$ | 6.3 | 12.5 | 3.1 |
| RANGE | (3.1-12.5) | (6.3-25) | (3.1-6.3) |
| 2q | | | |
| $MIC_{50}$ | 3.1 | 3.1 | 3.1 |
| RANGE | (1.6-3.1) | (3.1-6.3) | (1.6-3.1) |
| Vancomycin | | | |
| $MIC_{50}$ | 3.1 | 3.1 | 3.1 |
| RANGE | (1.6-3.1) | (1.6-6.3) | (3.1-6.3) |
| Teicoplanin | | | |
| $MIC_{50}$ | 1.6 | 6.3 | 0.4 |
| RANGE | (0.8-3.1) | (3.1-12.5) | (0.4-0.8) |

In Table 3 are shown data from standard microtiter assays carried out in the presence of no serum and 50% human serum. The test organisms were S. aureus, (1 and 2) S. epidermidis, (3 and 4) S. hemolyticus, (5) and S. faecalis, (6-8). These results show that the antibiotics of this invention are active in the presence of human serum.

Table 3

| Organism | Conditions | O-(ß-D-Glucosyl)-HPB-4 | Vancomycin | Teichloplanin |
|---|---|---|---|---|
| 1 | No serum | 1.6 | 1.6 | 1.6 |
| | 50% human serum | 3.1 | 3.1 | 6.3 |
| 2 | No serum | 3.1 | 3.1 | 1.6 |
| | 50% human serum | 3.1 | 6.3 | 6.3 |
| 3 | No serum | 3.1 | 3.1 | 1.6 |
| | 50% human serum | 6.3 | 6.3 | 3.1 |
| 4 | No serum | 6.3 | 3.1 | 3.1 |
| | 50% human serum | 12.5 | 6.3 | 3.1 |
| 5 | No serum | 12.5 | 3.1 | 12.5 |
| | 50% human serum | 50.0 | 12.5 | 50.0 |
| 6 | No serum | 1.6 | 3.1 | 0.8 |
| | 50% human serum | 3.1 | 12.5 | 6.3 |
| 7 | No serum | 1.6 | 3.1 | 0.8 |
| | 50% human serum | 1.6 | 6.3 | 6.3 |
| 8 | No serum | 1.6 | 6.3 | 0.8 |
| | 50% human serum | 1.6 | 12.5 | 3.1 |

The in vivo activity of the antibiotics of this invention was confirmed by administering selected antibiotics to mice infected with various gram-positive pathogens. Table 4 outlines the results comparing O-(β-D-Glucosyl)-HPB-4′ with Vancomycin and Teichoplanin against methicillin reistant (MR) and methicillin sensitive (MS) strains. Mice were injected i.p. with the indicated organism and then i.v., 1 hour post infection, with the indicated antibiotic.

Table 4

| Organisms Strain No. | Sensitivity to Methicillin | Vancomycin | $ED_{50}$ mg/kg Teichoplanin | O-(β-D-Glucosyl) HPB-4' |
|---|---|---|---|---|
| S. aureus HH 127 | MS | 0.8 | 0.8 | 0.8 |
| S. aureus 2620 | MR | 4.4 | 1.6 | 3.8 |
| S. faecalis 34358 | MR | 5.8 | 1.6 | 2.9 |
| S. epidermidis 2479 | MR | 13 | 26 | 23 |
| S. hemolyticus 651 | MR | 10 | 46 | > 60 |

The invention includes within its scope, pharmaceutical compositions containing at least one of the above-mentioned antibiotic compounds and a pharmaceutical acceptable carrier. The compositions may also contain other active antibacterial agents. The compositions may be made up in any pharmaceutical form appropriate for the route of administration in question. Such compositions are exemplified by solid compositions for oral administration, such as tablets, capsules, pills, powders and granules; liquid compositions for oral administration such as solutions, suspensions, syrups and elixirs; preparations for parenteral administration such as sterile solutions, suspensions or emulsions; and preparations for topical administration such as gels, creams, ointments of salves.

Effective injectable compositions containing these compounds may be in either suspension or solution form. In the preparation of suitable formulations it will be recognized that, in general, the water solubility of the acid addition salts is greater than that of the free bases. Similarly, the bases are more soluble in dilute acids or in acidic solutions than in neutral or basic solutions.

In the solution form the compound is dissolved in a physiologically acceptable vehicle. Such vehicles comprise a suitable solvent, preservatives such as benzyl alcohol, if needed, and buffers. Useful solvents include, for example, water and aqueous alcohols, glycols, and carbonate esters such as diethyl carbonate. Such aqueous solutions contain, in general, no more than 50% of the organic solvent by volume.

Injectable suspension compositions require a liquid suspending medium, with or without adjuvants, as a vehicle. The suspending medium can be, for example, aqueous polyvinylpyrrolidone, inert oils such as vegetable oils or highly refined mineral oils, or aqueous carboxymethylcellulose.

Suitable physiologically acceptable adjuvants may be necessary to keep the compound suspended in suspension compositions. The adjuvants may be chosen from among thickeners such as carboxymethylcellulose, polyvinylpyrrolidone, gelatin, and the alginates. Many surfactants are also useful as suspending agents. Lecithin, alkylphenol polyethylene oxide adducts, naphthalenesulfonates, alkylbenzenesulfonates, and the polyoxyethylene sorbitan esters are useful suspending agents.

Many substances which affect the hydrophilicity, density, and surface tension of the liquid suspending medium can assist in making injectable suspensions in individual cases. For example, silicone antiforms, sorbitol, and sugars can be useful suspending agents. A preferred antibacterial composition of the invention comprises O-(β-D-glucopyranosyl)-HPB-4′ in solutions for intramuscular or intravenous administration.

For use as an antibacterial agent, the compositions are administered so that the concentration of the active ingredient is greater than the minimum inhibitory concentration for the particular organism treated. The antibiotic compounds of the invention are effective in preventing and treating infection in an animal, including a human, by Gram-positive pathogenic bacteria. A typical parenteral dosage such as by intramuscular injections, for a 70 kg person, is about 100 to about 2000 mg, preferably about 500 to about 1000 mg, per day, although the optimum dosage will, of course, depend on factors such as the nature and severity of the bacterial infection, the animal and the route of admimistration. Optimum dosages can be determined readily by use of standard techniques. Once a day administration is preferred, though bid and tid administrastion is possible.

Certain antibiotics of this invention were also shown to have activity as animal growth promotants and as animal feed utilization efficiency enhancers. For increasing feed-utilization efficiency and promoting growth, a

compound of this application is administered orally in a suitable feed in an amount of from about 1 to about 200 grams per ton of total feed. For enhancing milk production in ruminants, oral administration of a daily amount of from about 0.1 to about 10 mg/kg of body weight is suggested.

The feed compositions of this invention comprise the normal feed rations of the meat and milk producing animals supplemented by a quantity of an active ingredient selected from among the antibiotics of formula I, and their salts, or a mixture thereof which is effective for improving the growth rate and feed efficiency of the animals but which is not toxic or noxious to a degree that the animals will reduce ingestion of the ration. The quantity of the active ingreident will vary, as is known to the art, with factors such as the cost of the ingredient, the species and the size of animal, the relative activity of the antibiotic selected or the type of feed ration used as the basal feed.

Representative feed rations for swine and poultry are as follows:

A swine ration for growing hogs of 18-45 kilos body weight is prepared using the following formula:

| | |
|---|---|
| Corn, ground | 78.15% |
| Soybean oil meal, 44% | 17.0% |
| Meat scraps, 50% | 3.0% |
| Oyster shell flavor | 0.4% |
| Bone meal | 0.5% |
| Zinc oxide | 0.1% |
| Vitamin A, B, $B_{12}$ & D supplement | optional |

A chicken ration for broilers is prepared using the following formula:

| | |
|---|---|
| Yellow corn meal | 67.35% |
| Soybean oil meal | 24.00% |
| Menhaden fish meal | 6.00% |
| Steamed bone meal | 1.00% |
| Ground limestone | 1.00% |
| Iodized salt | 0.34% |
| 25% choline chloride | 0.13% |
| Vitamin $B_{12}$ | 0.10% |
| Maganese sulfate | 0.02% |
| Vitamin mix | 0.06% |

Swine feed from weanling to fattening or finishing rations may be supplemented, Swine eat from about 0.9 kilogram of ration per day (for a 11 kg. pig) to 4 kilograms per day (for a 68 kilogram pig). Most rations are comprised of a corn base supplemented with legume silage, wheat bran, oats, barley, molasses or a protein supplement.

Poultry feeds comprise starter rations, broiler rations and laying rations. The rations are usually based on ground corn, corn meal or soybean meal. The broiler rations often contain high energy supplements such as added fats, proteins and vitamins. Turkey rations are similar, but comprise only a starting ration and a growing ration. Chickens or pheasants eat from 13.5-135 grams of feed per day, turkeys twice that much. Estimated intake of feed is dependent on the weight and age of the meat producing animal.

The active ingredients selected from among the antibiotics of formula I or a mixture thereof are mixed uniformly with such feed rations to give supplemented rations which are then fed as to custom, which is, most often, ad libitum. Conveniently, to do this, a premix of the supplemental growth promotant of this invention, optionally combined with or without other supplements known to this art such as an anthelmintic, a nitrogen source or an antibiotic, for example, virginiamycin or oxytetracycline is prepared by the manufacturer for sale to the formulators or feed lot operators. the concentration of the active ingredients selected from among the antibiotics of formula I or a mixture thereof in the premix is usually from 5-75% by weight or a concentration 100-2000 times greater than that in the complete feed ration. The premix form may be liquid or solid. Premix vehicles are corn oil, cottonseed oil, molasses or distillers solubles to form a liquid premix preparation. Sucrose, lactose, corn meal, ground corn, flour, calcium carbonate or soybean meal are often used as bases for solid premix preparations. The premix composition is, then, moxed uniformly with whole ration which is fed to the target animal. Such premix compositions are included in the term "feed compositions" as used herein.

The concentration of the active ingredients selected from among the antibiotics of formula I or a mixture thereof in the complete ration is a nontoxic but active quantity chosen, for example, from a range of about 1-1000 parts of active ingredient by weight per million parts of whole feed (ppm) or about 2-115 grams per metric ton. Advantageously, a nontoxic quantity of active ingredient is chosen from the range of 10-50 ppm.

The method of this invention comprises feeding to monogastric or ruminant, meat or milk producing animals, especially beef and dairy cattle, sheep, swine and poultry, an effective growth promoting but nontoxic quantity of an active ingredient selected from among the antibiotics of formula I. Other monogastric animals whose digestive tract also features fermentation in a cecum or cecum-like chamber are rabbits and horses.

The supplemented feed rations, described above, are presented in the animal by methods known to the art. Ad libitum feeding in the pasture, pen or growing shed is most convenient to increase the growth and milking

rate of the animal and to increase the feed efficiency of the operation.

The following examples are illustrative, and not limiting of this invention.

EXAMPLE 1

Preparation of O-(2,3,4,6-tetra-O-acetly-β-D-glucopyranosyl)-HPB-4′

740 mg (1.8 mmoles) of 2,3,4,6-tetra-O-acetyl-D-glucopyranosyl bromide (α-acetobromoglucose) in 1.4 ml of acetone was rapidly added to a solution of 545 mg of HPB-4′ (91% pure by HPLC) in 2.8 ml acetone, 1.8 ml water and 2.5 ml (0.703M, 1.76 mmoles) potassium hydroxide solution. The solution was stirred for two hours at room temperature. The acetone was removed in vacuo. The remaining solution contained O-(2,2,4,6-tetra-O-acetyl-β-D-glucopyranosyl)-HPB-4′.

EXAMPLE 2

Preparation of O-(β-D-glucopyranosyl)-HPB-4′

A crude mixture of O-(2,3,4,6-tetra-O-acetyl-β-D-acetylglucopyranosyl)-HPB-4′ prepared substantially as in Example 1, was treated with 10 ml of methanol/concentrated ammonium hydroxide (1:1) for three hours at room temperature to remove the sugar protecting groups. The solvents were removed in vacuo. The crude product mixture containing O-(β-D-glucopyranosyl)-HPB-4′ was suspended in aqueous sodium phosphate (0.04 M, pH 7.0); the pH was adjusted to ca. 8 with ammonium hydroxide to effect homogeneity. The filtered solution was placed on a column of D-Ala-D-Ala affinity gel. The column-bound glycopeptide was washed with sodium phosphate (0.04 M and 0.02 M, pH 7.0); and water, and the bound material eluted with 50% acetonitrile in aqueous ammonia (0.1 M).

The affinity-isolated material was purified by semi-preparative reversed phase HPLC on Whatman Partisil®-10 ODS packing in a steel column using an isochratic system of acetonitrile (12%) in potassium phosphate (0.01M, pH 6.0). Like fractions were pooled, diluted to 5-10% organic solvent and loaded onto a column of HP-20 (DIAION®) resin. The product was washed with 10 column volumes of water and then eluted with 50% aqueous acetonitrile. The acetonitrile was removed in vacuo and the water by lyophilization to give 230 mg of the title compound (41% yield); having a mass +1 of 1453, $E^{1\%}$ of 53 and pI of 7.9.

Chemical shift assignments in the 500 MHz $^1H$ NMR spectrum were made by analogy to those observed for the parent, HPB-4, and were confirmed by several homode-coupling experiments. NOE difference experiments then were conducted. Irradiation of Sg 1 produced NOE's to the other glucose resonances and to C5, which is consistent only with B4 glucosylation. The large coupling constant at Sg 1 (δ 5.49, J = 8.6 Hz) confirms the β-linkage.

EXAMPLE 3

HPLC Analytical Separation of O-(2,3,4,6-tetra-O-acetyl-β-D-glucopyranosyl)-HPB-4′

O-(2,3,4,6-tetra-O-acetyl-β-D-glucopyranosyl)-HPB-4′ prepared substantially as in Example 1 was separated using the following conditions:

Column: 4.6 × 150 mm Altex ODS 5 µm

Mobile phase: 0.01M phosphate buffer (pH 3.2); 14% acetonitrile with gradient to 37% in 8 minutes 1.5 ml per minute

Detection: 220 nm, 0.05 AUFS

O-(2,3,4,6-tetra-O-acetylglucopyranosyl)-HPB-4′ had a retention time of 4.9 min.

EXAMPLE 4

HPLC Analytical Separation of O-(β-D-glucopyranosyl)-HPB-4′

O-(β-D-glucopyranosyl)-HPB-4′ prepared substantially as in example 2 was separated using the following conditions:

Column: 4.6 × 150 mm Altex ODS 5 µm

Mobile phase: 0.01 M phosphate buffer (pH 3.2), 7% acetonitrile with gradient to 34% in 12 minutes. 1.5 ml per minute.

Detection: 220 nm, 0.05 AUFS

O-(β-glucopyranosyl)HPB-4′ had a retention time of 8.4 min.

EXAMPLES 5-28

Using substantially the procedures of both Examples 1 and 2, the compounds of Example 5 to 28 were obtained by using the appropriate glycopeptide and protected α-halo sugar starting materials. The protecting groups, base and reaction conditions remained substantially the same with only the time needed to remove the protecting group changed as indicated in the table.

| Example No. | Compound | Deprotection Reaction time in hrs. | Low Resolution FAB MS $MH^+$ | $E_{1\%}$ | pI |
|---|---|---|---|---|---|
| 5 | O-(ß-D-mannopyranosyl)-AAD-216 aglycone | 3 | 1458 | 61 | 5.1 |
| 6 | O-(2-acetamido-2-deoxy-ß-D-glucopyranosyl)-AAD-216 aglycone | 15 | 1499 | 63 | 4.9 |
| 7 | O-(2-amino-2-deoxy-ß-D-glucopyranosyl)-AAD-216 aglycone | 17 | 1457 | 67 | 6.9 |
| 8 | O-(6-amino-6-deoxy-ß-D-glucopyranosyl)-AAD-216 aglycone | 17 | 1457 | 62 | 7.2 |
| 9 | O-(6-acetamido-6-deoxy-ß-D-glucopyranosyl-HPB-3' | 17 | 1499 | - | 5.1 |
| 10 | O-(ß-D-maltosyl)-AAD-216 aglycone | 17 | 1620 | 57 | 5.1 |
| 11 | O-(2-acetamido-2-deoxy-ß-D-glucopyranosyl)-AAD-216 mannosylaglycone | 6 | 1661 | 55 | 5.1 |
| 12 | O-(ß-D-glucopyranosyl)-AAD-216 aglycone | 3 | 1458 | 68 | 5.1 |
| 13 | O-(2-amino-2-deoxy-ß-D-glucopyranosyl)-CWI-785B' aglycone | 9 | 1323 | 55 | 7.3 |
| 14 | O-(ß-D-glucopyranosyl)-HPB-3' | 2 | 1469 | 42 | 7.9 |
| 15 | O-(2-amino-2-deoxy-ß-D-glucopyranosyl)-HPB-4' | 15 | 1452 | 53 | 7.9 |
| 16 | O-(6-amino-6-deoxy-ß-D-glucopyranosyl)AAJ-271 mannosyl aglycone | 6 | 1535 | 64 | 7.2 |
| 17 | O-[2-(benzyloxycarbonyl)amino-2-deoxy-ß-D-glucopyranosyl]-HPB-4' | 3 | 1586 | 46 | 7.7 |
| 18 | O-(2-butanamido-2-deoxy-ß-D-glucopyranosyl)-HPB-4' | 3 | 1522 | 46 | 7.7 |
| 19 | O-[2-(3-methylbutanamido)-2-deoxy-ß-D-glucopyranosyl]-HPB-4' | 3 | 1536 | 46 | 7.7 |
| 20 | O-[2-(2-methylpropoxycarbonyl)amino-2-deoxy-ß-D-glucopyranosyl]-HPB-4' | 3 | 1552 | 46 | 7.9 |

| Example No. | Compound | Deprotection Reaction time in hrs. | Low Resolution FAB MS $MH^+$ | $E_{1\%}$ | pI |
|---|---|---|---|---|---|
| 21 | O-[2-(1-methylethoxycarbonyl)amino-2-deoxy-ß-D-glucopyranosyl)-HPB-4' | 3 | 1518 | 50 | 7.8 |
| 22 | O-[2-(methoxycarbonyl)amino-2-deoxy-ß-D-glucopyranosyl]-HPB-4' | | 1510 | 52 | 7.9 |
| 23 | O-(2-acetamido-2-deoxy-ß-D-glucopyranosyl)-HPB-4' | 3 | 1494 | 49 | 7.8 |
| 24 | O-(2-amino-2-deoxy-ß-D-gluco-pyranosyl)vancomycin aglycone | 16 | 1304 | 50 | 7.5 |
| 25 | -(6-amino-6-deoxy-ß-D-gluco-pyranosyl)vancomycin aglycone | 16 | 1304 | 47 | 7.9 |
| 26 | O-[2-(propoxycarbonyl)amino-2-deoxy-ß-D-glucopyranosyl]-HPB-4' | 3 | 1538 | 53 | 7.8 |
| 27 | O-[2-(ethoxycarbonyl)amino-2-deoxy-ß-D-glucopyranosyl]-HPB-4' | 2 | 1524 | 47 | 7.9 |
| 28 | O-(2-propanamido-2-deoxy-ß-D-glucopyranosyl)-HPB-4' | 3 | 1508 | 52 | 7.6 |

EXAMPLES 29-30

Examples 29 to 39 can be prepared using the following procedure with the appropriate glycopeptide and protected α-halo sugar starting materials. An acetone solution of the O-acetylated glycosyl halide (2 to 10 equivalents) is rapidly added to a solution of a glycopeptide (.02-.05 M) in aqueous acetone containing 2 to 10 equivalents of potassium hydroxide. The resulting solution is stirred for one to two hours at room temperature. The solvents are removed in vacuo. The residue is treated with concentrated ammonium hydroxide in methanol (1:1) for 1 to 30 hours at room temperature to cleave the protecting groups.

Chromatographic purification is conducted using a D-Ala-D-Ala affinity gel column and reversed phase HPLC.

| Example No. | Compound |
|---|---|
| 29 | O-(ß-D-ribofuranosyl)-HPB-4' |
| 30 | O-(ß-D-ribopyranosyl)-HPB-4' |
| 31 | O-(ß-L-rhamnopyranosyl)-HPB-4' |
| 32 | O-(2-deoxy-ß-D-glucopyranosyl)-HPB-4' |
| 33 | O-[2-(methylsulfonyl)amino-2-deoxy-ß-D-glucopyranosyl]-HPB-4' |
| 34 | O-[2-(phenylsulfonyl)amino-2-deoxy-ß-D-glucopyranosyl]-HPB-4' |
| 35 | O-[2-[(methylamino)carbonyl]amino-2-deoxy-ß-D-glucopyranosyl] HPB-4' |
| 36 | O-[2-[(phenylamino)carbonyl]amino-2-deoxy-ß-D-glucopyranosyl] HPB-4' |
| 37 | O-[2-(aminocarbonyl)amino-2-deoxy-ß-D-glucopyranosyl]-HPB-4' |
| 38 | O-(2-hexanamido-6-amino-2,6-dideoxy-ß-D-glucopyranosyl)-vancomycin aglycone |
| 39 | O-[2-(10-methylundecanamido)-2-deoxy-ß-D-glucopyranosyl] |

EXAMPLE 40

O-[2-(1-Methylpent-4-ene-amino)-2-deoxy-β-D-glucopyranosyl]-AAD-216 aglycone

A solution of AAD-216 aglycone (800 mg) in dry DMF (20 ml) is treated with di-t-butyldicarbonate (172 μl) and triethylamine (285 μl) for one hour at room temperature. The DMF is removed in vacuo and the residue is treated with 7.5 ml of 7.5 N ammonium hydroxide in 7.5 ml methanol for 3 hours. The liquid is removed in vacuo. The residue (645 mg) is taken up in aqueous potassium hydroxide (4.5 ml, 0.62 M) and acetone (8 ml) and is stirred at room temperature while a solution of 2-trifluoroacetamido-2-deoxy-3,4,6-tri-O-acetyl-α-D-glucopyranosyl bromide (1.3 g) in acetone (4 ml) is rapidly added. After two hours, the liquids are removed. The residue is treated with a solution of 1:1 concentrated ammonium hydroxide/methanol (25 ml) for 18 hours and the liquids are removed in vacuo. The residue is suspended in a sodium phosphate solution (0.04 M) and the pH is adjusted to about 8.5 with ammonium hydroxide to effect homogeneity. The solution is filtered and partially purified using D-Ala-D-Ala affinity gel.

A solution of the partially purified O-(2-amino-2-deoxy-β-D-glucopyranosyl)-N-(t-butyloxycarbonyl)-AAD-216 aglycone and excess 5-hexene-2-one in absolute ethanol is stirred at room temperature under nitrogen for 24 hours. Excess sodium borohydride is added and the mixture is allowed to stir for an additional 5 hours. The mixture is diluted with water and concentrated to dryness. The residue is carefully treated with dry TFA for 5 minutes. The TFA is then removed in vacuo. The residue is purified using affigel, HPLC and HP-20 desalting to give O-[2-(1-methylpent-4-ene-amino)-2-deoxy-β-D-glucopyranosyl]-AAD-216 aglycone.

## Claims

1. A compound of the formula:

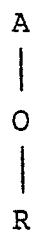

wherein A is the remaining portion of a glycopeptide antibiotic of the vancomycin/ristocetin class or a chemical derivative thereof; and O is the phenolic oxygen of the central (B) ring of the triaryl ether region of the glycopeptide antibiotic; and R is a saccharide selected from: monosaccharides, disaccharides, and their deoxy and amino variants, provided R is not the natural sugar species attached at this site on the glycopeptide core, and R is not glucosyl when A is the remaining portion of vancomycin, A51568A, M43D, or A51568B; or a pharmaceutically acceptable salt thereof.

2. A compound of claim 1 wherein A is the remaining portion of a glycopeptide of the vancomycin/ristocetin class selected from:

vancomycin
ristocetin
actaplanin
A 35512B
teicoplanin
AAJ-271 glycopeptides
A41030 factors a, b, c, d, e, f, g
CWI-785 glycopeptides
AM 374
actinoidin
A 477
AAD-216 glycopeptides
OA 7653
avoparcin;

or their hydrolysis products, N-acyl or ester derivatives.

3. A compound of claim 1 wherein A is the remaining portion of a glycopeptide antibiotic of the vancomycin/ristocetin class selected from: AAD-216 aglycone, AAD-216 mannosyl aglycone, HPB-4′, HPB-3′, CWI-785B′-aglycone, AAJ-271-mannosylaglycone, and vancomycin aglycone.

4. A compound of claim 1 wherein A is the remaining portion of HPB-4′.

5. A compound of any one of claims 1 to 4 wherein R is a monosaccharide or its deoxy or amino variant.

6. A compound of any one of claims 1 to 4 wherein R is a disaccharide of its deoxy or amino variant.

7. A compound of claim 5 wherein R is a pyranosyl monosaccaride or its deoxy or amino variant.

8. A compound of claim 5 wherein R is a furanosyl monosaccaride or its deoxy or amino variant.

9.A compound of any one of claim 1 to 4 in which R is selected from glucosyl, amino-deoxy-glucosyl, diamino-dideoxyglucosyl, N-acylamino-deoxy-glucosyl or compounds of the formula:

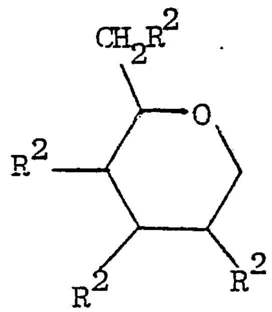

wherein
$R^2$ is OH, $NR^3R^4$, or $NR^3$-$(CH_2)_s$-$W_p$-$(CH_2)_qR^5$;
$R^3$ is H or $C_1$-$C_4$ alkyl;
$R^4$ is H;

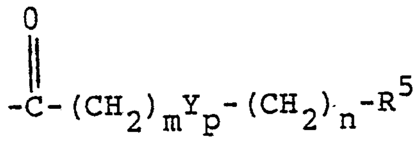, or

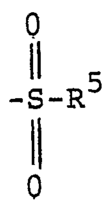, or

p is 0 or 1;
m and n are integers from 0 to 4;
s is an integer from 2 to 4;
q is an integer from 1 to 4;
$R^5$ is H, $C_1$-$C_4$-alkyl, $C_3$-$C_8$-cycloalkyl, $C_4$-$C_8$-cycloalkenyl, or phenyl;
W is $CH_2$, -C≡C- -CH=CH-, -$C(CH_3)$=CH-, -CH=$C(CH_3)$- or -$C(CH_3)$=$C(CH_3)$;-
Y is $CH_2$, O, -N($C_1$-$C_4$-alkyl)-, -NH-, -C≡C-, -CH=CH-, -$C(CH_3)$=CH-, -CH=$C(CH_3)$- or -$C(CH_3)$=$C(CH_3)$-;
and wherein $R^5$ and the connecting alkyl groups -$(CH_2)_m$-, -$(CH_2)_n$-, -$(CH_2)_s$- and -$CH_2)_q$- are optionally substituted by one or two halo, methyl, ethyl, methoxy, amino, N-protected-amino, methylamino, dimethylamino, acetamido, or hydroxyl groups, provided that $R^5$ is not H when Y is O, p is 1, m is O and n is O; and provided that at least two $R^2$ groups are OH.

10. A compound of any one of claims 1 to 4 in which R is glucosyl, 2-acetamido-2-deoxy-D-glucosyl, 2-(1-methylethoxycarbonyl)amino-2-deoxy-D-glucosyl, 2-(ethoxycarbonyl)amino-2-deoxy-D-glucosyl or, 2-(methoxycarbonyl)amino 2-deoxy-D-glucosyl.

11. A compound of claim 1, selected from the group consisting of:

O-(β-D-glucopyranosyl)-AAD-216 aglycone
O-(β-D-mannopyranosyl)-AAD-216 aglycone
O-(2-acetamido-2-deoxy-β-deoxy-β-D-glucopyranosyl)-AAD-216 aglycone
O-(2-amino-2-deoxy-β-D-glucopyranosyl)-AAD-216 aglycone
O-(6-amino-6-deoxy-β-D-glucopyranosyl)-AAD-216 aglycone
O-(β-D-maltosyl)-AAD-216 aglycone
O-(2-acetamido-2-deoxy-β-D-glucopyranosyl)-AAD-216-mannosylaglycone
O-(2-amino-2-deoxy-β-D-glucopyranosyl)-CWI-785B′ aglycone O-(β-D-glucopyranosyl)-HPB-3′
O-(2-amino-2-deoxy-β-D-glucopyranosyl)-HPB-4′
O-(6-amino-6-deoxy-β-D-glucopyranosyl)-AAJ-271 mannosylaglycone
O-(2-amino-2-deoxy-β-D-glucopyanosyl)-vancomycinaglycone
O-(6-amino-6-β-D-glucopyranosyl)-vancomycinaglycone
O-(6-acetamido-6-deoxy-β-D-glucopyranosyl)-AAD-216 aglycone
O-[2-(benzyloxycarbonyl)amino-2-deoxy-β-D-glucopyranosyl]-HPB-4′

O-(2-butanamido-2-deoxy-β-D-glucopyranosyl)-HPB-4′
O-[2-(3-methylbutanamido)-2-deoxy-β-D-glucopyranosyl]-HPB-4′
O-[2-(2-methylpropoxycarbonyl)amino-2-deoxy-β-D-glucopyranosyl]-HPB-4′
O-(2-propanamido-2-deoxy-β-D-glucpyrasnosyl)-HPB-4′.
O-(2-acetamido-2-deoxy-β-D-glucopyranosyl)-HPB-4′
O-[2-(1-methylethoxycarbonyl)amino-2-deoxy-β-D-glucopyranosyl]-HPB-4′
O-[2-(methoxycarbonyl)amino-2-deoxy-β-D-glucopyranosyl]-HPB-4′.

12. A compound of claim 1 which is O-(β-D-glucopyranosyl)-HPB-4′.
13. A compound of claim 1 which is O-[2-(ethoxycarbonyl)amino-2-deoxy-β-D-glucopyranosyl]-HPB-4′.
14. A process for preparing a compound of the formula

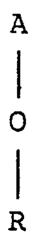

or a chemical derivative thereof, or a pharmaceutically acceptable salt thereof, wherein A is the remaining portion of a glycopeptide antibiotic of the vancomycin/ristocetin class or a chemical derivative thereof, and O is the phenolic oxygen of the central (B) ring of the triaryl ether region of the glycopeptide antibiotic and R is a saccharide selected from: monosaccharides, disaccharides, and their deoxy and amino variants which process comprises reacting a protected glycosyl halide with an alkaline solution of a glycopeptide antibiotic of the formula A-O-H or a chemical derivative thereof, wherein A and O are as hereinbefore defined, for a time sufficient to produce the protected glycosylated glycopeptide antibiotic, and thereafter;

i) deprotecting the functionalities of the added glycosyl group,
ii) optionally forming a chemical derivative thereof,
iii) optionally a pharmaceutically acceptable salt thereof.

15. A pharmaceutically composition which comprises a compound according to any one of claims 1 to 13 and a pharmaceutically acceptable carrier.
16. A compound according to any one of claims 1 to 13 for use as a medicament.
17. A feed composition which comprises a compound of any one of claims 1 to 13, or a pharmaceutically acceptable salt or any mixture thereof, and a standard feed ration.
18. A method for increasing feed utilization in a domesticated animal which comprises orally administering to the domesticated animal a compound or composition of any one of claims 1 to 13, 15 or 17 or any mixture thereof.
19. A method of improving milk production in a lactating ruminant comprising orally administering to the ruminant a compound or a composition of any one of claims 1 to 13, 15 or 17 or any mixture thereof.

Claims for the following Contracting States: GR and ES.

1. a process for preparing a compound of formula I

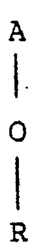

(I)

or a chemical derivative thereof, or a pharmaceutically acceptable salt thereof, wherein A is the remaining portion of a glycopeptide antibiotic of the vancomycin/ristocetin class or a chemical derivative thereof, and O is the phenolic oxygen of the central (B) ring of the triaryl ether region of the glycopeptide antibiotic and R is a saccharide selected from: monosaccharides, disaccharides, and their deoxy and amino variants; which process comprises reacting a protected gylcosyl halide with an alkaline solution of a glycopeptide antibiotic of the formula A-O-H or a chemical derivative thereof, wherein A and O are as hereinbefore defined, for a time sufficient to produce the protected glycosylated glycopeptide antibiotic, and thereafter;

i) deprotecting the functionalities of the added glycosyl group,
ii) optional forming a chemical derivative thereof,
iii) optionally forming a pharmaceutically acceptable salt thereof.

2. The process according to claim 1 wherein A is the remaining portion of a glycopeptide of the vancomycin/ristocetin class selected from:

vancomycin
ristocetin
actaplanin
A 35512B
teicoplanin
AAJ-271 glycopeptides
A41030 factors a, b, c, d, e, f, g
CWI-785 glycopeptides
AM 374
actinoidin
A 477
AAD-216 glycopeptides
OA 7653
avoparcin;

or their hydrolysis products, N-acyl or ester derivatives.

3. The process according to claim 1 wherein A is the remaining portion of a glycopeptide antibiotic of the vancomycin/ristocetin class from: AAD-216 aglycone, AAD-216 mannosyl aglycone, HPB-4′, HPB-3′, CWI-785B′-aglycone, AAJ-271-mannosylaglycone, and vancomycin aglycone.

4. The process according to claim 1 wherein A is the remaining portion of HPB-4′.

5. The process according to any one of claims 1 to 4 wherein R is a monosaccharide or its deoxy or amino variant.

6. The process according to any one of claims 1 to 4 wherein R is a disaccharide or its deoxy or amino variant.

7. The process; according to claim 5 wherein R is a pyranosyl monosaccharide or its deoxy or amino variant.

8. The process according to claim 5 wherein R is a furanosyl monosaccharide or its deoxy or amino variant.

9. The process according to any one of claims 1 to 4 in which R is selected from glucosyl, amino-deoxyglucosyl, diamino-dideoxyuglucosyl, N-acylamino-deoxyglucosyl or compounds of the formula:

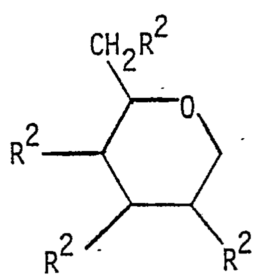

wherein
$R^2$ is OH, $NR^3R^4$, or $NR^3$-$(CH_2)_s$-$W_p$-$(CH_2)_qR^5$;
$R^3$ is H or $C_1$-$C_4$ alkyl;
$R^4$ is H;

O
||
$-C-(CH_2)_mY_p-(CH_2)_n-R^5$, or

O
||
$-S-R^5$, or
||
O

p is 0 to 1;
m and n are integers from 0 to 4;
s is an integer from 2 to 4;
q is an integer from 1 to 4;
$R^5$ is H, $C_1$-$C_4$-alkyl, $C_3$-$C_8$-cycloalkyl, $C_4$-$C_8$-cycloalkenyl, or phenyl;
W is $CH_2$, $-C{\equiv}C-$, $-CH=CH-$, $-C(CH_3)=CH-$, $-CH=C(CH_3)-$ or $-C(CH_3)=C(CH_3)$;-
Y is $CH_2$, O, -N($C_1$-$C_4$ alkyl)-, -NH-, $-C{\equiv}C-$, $-CH=CH-$, $-C(CH_3)=CH-$, $-CH=C(CH_3)-$ or $-C(CH_3)=C(CH_3)-$;
and wherein $R^5$ and the connecting alkyl groups $-(CH_2)_m-$, $-(CH_2)_n-$, $-(CH_2)_s-$ and $-CH_2)_q-$ are optionally substituted by one or two halo, methyl, ethyl, methoxy, amino, N-protected-amino, methylamino, dimethylamino, acetamido, or hydroxyl groups, provided that $R^5$ is not H when Y is O, p is 1, m is O and n is O; provided that at least two $R^2$ groups are OH.

10. The process according to any one of claims 1 to 4 in which R is glucosyl, 2-acetamido-2-deoxy-D-glucosyl, 2-(1-methylethoxycarbonyl)amino-2-doexy-D-glucosyl, 2-(ethoxycarbonyl)amino-2-deoxy-D-glucosyl or, 2-(methoxycarbonyl)amino 2-deoxy-D-glucosyl.

11. The process according to claim 1, for preparing a compound which is:

O-(β-D-glucopyranosyl)-AAD-216 aglycone
O-(β-D-mannopyranosyl)-AAD-216 aglycone
O-(2-acetamido-2-deoxy-β-D-glucopyranosyl)-AAD-216 aglycone
O-(2-amino-2-deoxy-β-D-glucopyranosyl)-AAD-216 aglycone
O-(6-amino-6-deoxy-β-D-glucopyranosyl)-AAD-216 aglycone
O-(β-D-maltosyl)-AAD-216 aglycone
O-(2-acetamido-2-doexy-β-D-glucopyranosyl)-AAD-216-mannosylaglycone
O-(2-amino-2-deoxy-β-D-glucopyranosyl)-CWI-785B′ aglycone
O-(β-D-glucopyranosyl)-HPB-3′
O-(2-amino-2-deoxy-β-D-glucopyranosyl)-HPB-4′
O-(6-amino-6-deoxy-β-D-glucopyranosyl)-AAJ-271 mannosylaglycone
O-(2-amino-2-deoxy-β-D-glucopyranosyl)-vancomycinaglycone
O-(6-amino-6-deoxy-β-D-glucopyranosyl)-vancomycinaglycone
O-(6-acetamido-6-deoxy-β-D-glucopyranosyl)-AAD-216 aglycone
O-[2-(benzyloxycarbonyl)amino-2-deoxy-β-D-glucopyranosyl]-HPB-4′
O-(2-butanamido-2-deoxy-β-D-glucopyranosyl)-HPB-4′
O-[2-(3-methylbutanamido)-2-deoxy-β-D-glucopyranosyl]-HPB-4′
O-[2-(2-methylpropoxycarbonyl)amino-2-deoxy-β-D-glucopyranosyl]-HPB-4′
O-(2-propanamido-2-deoxy-β-D-glucpyranosyl)-HPB-4′.
O-(2-acetamido-2-deoxy-β-D-glucopyranosyl)-HPB-4′
O-[2-(1-methylethoxycarbonyl)amino-2-deoxy-β-D-glucopyranosyl]-HPB-4′
O-[2-(methoxycarbonyl)amino-2-deoxy-β-D-glucopyranosyl]-HPB-4′ or a pharmaceutically acceptable salt thereof.

12. The process of claim 1 for preparing a compound which is O-(β-D-glucopyranosyl)-HPB-4′.

13. The process of claim 1 for preparing a compound which is O-[2-(ethoxycarbonyl)amino-2-deoxy-β-D-glucopyranosyl]-HPB-4′.

14. A process for preparing a pharmaceutical composition which comprises bringing into association a compound of formula (I) as defined in claim 1 or a pharmaceutically acceptable salt thereof and a pharmaceutically acceptable carrier.

15. A process for preparing an animal feed composition comprising the admixing of an animal feed with a product as defined in any one of claims 1 to 13, or a pharmaceutically acceptable salt thereof.

16. A method of improving the growth rate or feed efficiency of meat or milk producing animals which

comprises administering a product or composition as produced in any one of claims 1 to 15, or any mixture thereof.

17. A method of improving milk production in a lactating ruminant which comprises administering to an animal a product or composition as defined in any one of claims 1 to 15, or any mixture thereof.